# EUROPEAN PATENT APPLICATION

(11) **EP 4 098 746 A1**
(43) Date of publication of application: **07.12.2022**
(21) Application number: 21746318.1
(22) Date of filing: 29.01.2021
(51) Int. Cl.: C12N 15/113, C07F 9/6512, C07F 9/6561

(54) **SINGLE-STRANDED POLYNUCLEOTIDE**

(30) Priority: 31.01.2020 JP 2020015404
(71) Applicant: National University Corporation Tokai National Higher Education and Research System, Nagoya-shi, Aichi 464-8601 (JP)
(72) Inventor: ASANUMA, Hiroyuki, Nagoya-shi, Aichi 464-8601 (JP); KAMIYA, Yukiko, Nagoya-shi, Aichi 464-8601 (JP); SATO, Fuminori, Nagoya-shi, Aichi 464-8601 (JP); MURAYAMA, Keiji, Nagoya-shi, Aichi 464-8601 (JP); YOKOYAMA, Junya, Nagoya-shi, Aichi 464-8601 (JP)
(74) Representative: Hoffmann Eitle
(86) International application number: PCT/JP2021/003332
(87) International publication number: WO 2021/153762

(57) **Abstract**

An object is to provide a technique that can enhance the binding properties of a single-stranded polynucleotide comprising a palindromic structure comprising an acyclic polynucleotide structural unit to target polynucleotides, such as miRNA, while suppressing self-duplex formation of the single-stranded polynucleotide. This object is achieved by a single-stranded polynucleotide comprising a palindromic structure comprising an acyclic polynucleotide structural unit, wherein adenine in the palindromic structure is replaced by diaminopurine, and thymine at a position complementary to the adenine is replaced by a thiouracil derivative.

## Description

### Technical Field

The present invention relates to a single-stranded polynucleotide and the like.

### Background Art

Artificial polynucleotides, such as SNA (serinol nucleic acid) and aTNA (acyclic threoninol nucleic acid), have been reported to recognize DNA and RNA in a sequence-specific manner (NPL 1 and NPL 2). Since these are acyclic polynucleotides that do not have a sugar skeleton, they are highly resistant to enzymatic degradation in vivo. They are also said to be easily synthesized. Therefore, acyclic polynucleotides are expected to be used as anti-miRNA, anti-mRNA, and siRNA, as well as molecular beacons.

### Citation List

### Non-patent Literature

NPL 1: H. Kashida, K. Murayama, T. Toda, H. Asanuma, Angew. Chem. Int. Ed. 2011, 50, 1285-1288.
NPL 2: K. Murayama, H. Kashida, H. Asanuma, Chem. Commun., 2015, 51, 6500-6503.

### Summary of Invention

### Technical Problem

In the course of research, the present inventors found that acyclic polynucleotides comprising a palindromic structure represented by formula (2): A¹-B-A² (wherein A¹ and A² are base sequences complementary to each other, and B is any base sequence) form self-duplexes even through there is some mismatch, which causes a problem that they cannot bind to target polynucleotides (e.g., in vivo miRNA and mRNA).

Accordingly, an object of the present invention is to provide a technique that can enhance the binding properties of a single-stranded polynucleotide comprising a palindromic structure comprising an acyclic polynucleotide structural unit to target polynucleotides, such as miRNA, while suppressing self-duplex formation of the single-stranded polynucleotide. In another aspect, an object of the present invention is to provide a technique of improving the antisense activity of the single-stranded polynucleotide comprising a palindromic structure comprising an acyclic polynucleotide structural unit.

### Solution to Problem

As a result of extensive research, the present inventors found that the above objects can be achieved by a single-stranded polynucleotide comprising a palindromic structure comprising an acyclic polynucleotide structural unit, wherein adenine in the palindromic structure is replaced by diaminopurine, and thymine at a position complementary to the adenine is replaced by a thiouracil derivative.

In synthesis of an acyclic single-stranded polynucleotide comprising diaminopurine and/or a thiouracil derivative by a phosphoramidite method, various impurities remain when conventional amidite monomers of diaminopurines and thiouracil derivatives are used. As a result of further research, the present inventors found that this problem can be solved by employing protecting groups removable by acids as the protecting groups of bases in acyclic amidite monomers of diaminopurines and thiouracil derivatives.

As a result of further research based on these findings, the present inventors have completed the present invention. Specifically, the present invention includes the following embodiments.

### Item 1.

A single-stranded polynucleotide comprising a palindromic structure comprising an acyclic polynucleotide structural unit, wherein adenine and/or an analog thereof in the palindromic structure is replaced by diaminopurine, and thymine and/or an analog thereof at a position complementary to the adenine is replaced by a thiouracil derivative.

### Item 2.

The single-stranded polynucleotide according to Item 1, wherein the acyclic polynucleotide structural unit is represented by formula (1): 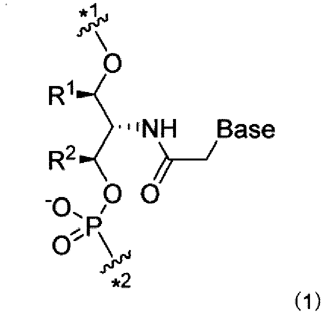 wherein R¹ and R² are the same or different, and each is a hydrogen atom or an organic group, excluding a case where R¹ and R² are both organic groups, and Base is a nucleobase.

### Item 3.

The single-stranded polynucleotide according to Item 2, wherein R¹ is a hydrogen atom or a chain hydrocarbon group, and R² is a hydrogen atom.

### Item 4.

The single-stranded polynucleotide according to any one of Items 1 to 3, wherein the palindromic structure is a single-stranded polynucleotide consisting of a base sequence represented by formula (2): A¹-B-A², wherein the base length of A¹ and A² is 3 or more, A¹ and A² are base sequences complementary to each other, and the base length of B is an integer of 0 to (base length of A¹ + base length of A²)/4).

### Item 5.

The single-stranded polynucleotide according to any one of Items 1 to 4, wherein the palindromic structure consists of the acyclic polynucleotide structural unit.

### Item 6.

The single-stranded polynucleotide according to any one of Items 1 to 5, wherein the number of diaminopurines and the number of thiouracil derivatives are each 2 or more.

### Item 7.

The single-stranded polynucleotide according to any one of Items 1 to 6, which has a base length of 6 to 500.

### Item 8.

The single-stranded polynucleotide according to any one of Items 1 to 7, which is complementary to a cellular endogenous polynucleotide.

### Item 9.

The single-stranded polynucleotide according to any one of Items 1 to 8, which is anti-miRNA or an anti-mRNA polynucleotide.

### Item 10.

A double-stranded polynucleotide comprising the single-stranded polynucleotide according to any one of Items 1 to 9.

### Item 11.

A compound or a salt thereof or a solvate thereof, the compound being represented by formula (3): 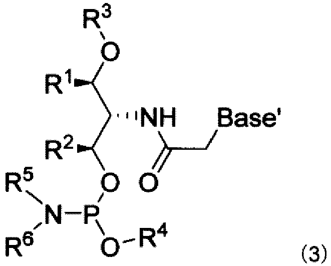 wherein R¹ and R² are the same or different, and each is a hydrogen atom or an organic group, excluding a case where R¹ and R² are both organic groups, R³ and R⁴ are the same or different, and each is a protecting group for a hydroxyl group, R⁵ and R⁶ are the same or different, and each is an alkyl group, and Base' is a nucleobase in which an amino group of diaminopurine is protected by a protecting group removable by an acid, or a nucleobase in which a thiocarbonyl group of a thiouracil derivative is protected by a protecting group removable by an acid.

### Item 12.

The compound or a salt thereof or a solvate thereof according to Item 11, wherein the compound is represented by formula (3A) or (3B): 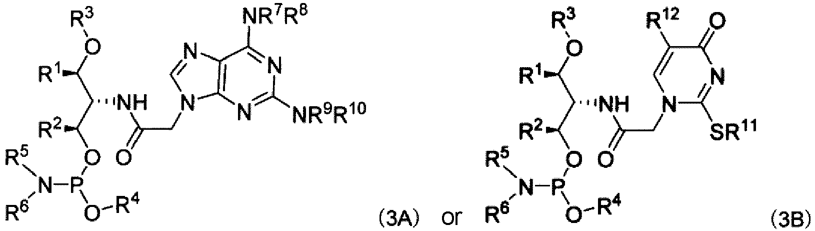 wherein R¹, R²/R³, R⁴, R⁵, and R⁶ are as defined above, R⁷, R⁸, R⁹, R¹⁰, and R¹¹ are the same or different, and each is a protecting group removable by an acid, and R¹² is a hydrogen atom or an alkyl group.

### Item 13.

The compound or a salt thereof or a solvate thereof according to Item 12, wherein R⁷, R⁸, R⁹, and R¹⁰ are Boc groups, and R⁹ is an MMPM group.

### Item 14.

The compound or a salt thereof or a solvate thereof according to any one of Items 11 to 13, wherein R³ is a group represented by formula (4): 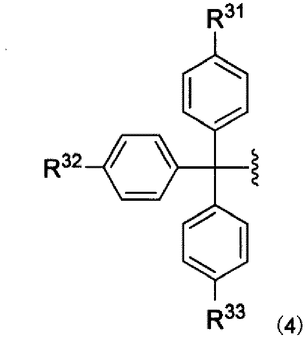 wherein R³¹, R³², and R³³ are the same or different, and each is a hydrogen atom or an alkoxy group;
R⁴ is -(CH₂)₂-CN; and
R⁵ and R⁶ are isopropyl groups.

### Item 15.

A method for producing a single-stranded polynucleotide by a phosphoramidite method using the compound or a salt thereof or a solvate thereof according to any one of Items 11 to 14 as an amidite monomer.

### Advantageous Effects of Invention

The present invention can provide a single-stranded polynucleotide comprising a palindromic structure comprising an acyclic polynucleotide structural unit that has higher binding properties to target polynucleotides, such as miRNA, while its self-duplex formation is suppressed. The present invention can also improve the antisense activity of the single-stranded polynucleotide comprising a palindromic structure comprising an acyclic polynucleotide structural unit. Further, the present invention can provide an amidite monomer that enables efficient synthesis of an acyclic single-stranded polynucleotide comprising diaminopurine and/or a thiouracil derivative, and a method for synthesizing the polynucleotide.

### Brief Description of Drawings

Fig. 1 shows the HPLC profiles of the SNA polynucleotides synthesized in Synthesis Example 3.
Fig. 2 shows the melting curves obtained in Test Example 1. The vertical axis shows the relative absorbance at 260 nm, and the horizontal axis shows temperature.
Fig. 3 shows the melting curves obtained in Test Example 2. The vertical axis shows the relative absorbance at 260 nm, and the horizontal axis shows temperature.
Fig. 4 shows the Native MS profile obtained in Test Example 2.
Fig. 5 shows the polyacrylamide gel electrophoretogram obtained in Test Example 2.
Fig. 6 shows the sequences of the single-stranded polynucleotides of Synthesis Example 4 and the results of Test Example 3. The gray areas indicate self-complementary regions.
Fig. 7 shows the results of Test Example 4. The vertical axis shows the relative luciferase activity. The leftmost bar of the horizontal axis shows a case where a control reporter plasmid (without a miR21-binding site) was introduced, and the other bars show cases where a reporter plasmid containing a miR21-binding site was introduced. In addition, the horizontal axis shows AMO names when introduced.
Fig. 8 shows the sequences of the single-stranded polynucleotides of Synthesis Example 5 and the results of Test Example 5. The gray areas indicate self-complementary regions.
Fig. 9 shows the sequences of the single-stranded polynucleotides of Synthesis Example 6 and the results of Test Example 6. The gray areas indicate self-complementary regions.
Fig. 10 shows the results of Test Example 7. The vertical and horizontal axes are the same as in Fig. 7.
Fig. 11 shows the sequences of the single-stranded polynucleotides of Synthesis Example 7 and the results of Test Example 8.
Fig. 12 shows the results of Test Example 9. The vertical and horizontal axes are the same as in Fig. 7.
Fig. 13 shows the sequences of the single-stranded polynucleotides of Synthesis Example 8 and the results of Test Example 10. The gray areas indicate self-complementary regions.
Fig. 14 shows the results of Test Example 11. The vertical and horizontal axes are the same as in Fig. 7.

### Description of Embodiments

In the present specification, the terms "comprise" and "contain" include the concepts of "comprise," "contain," "essentially consist of," and "consist of."

### 1. Single-Stranded Polynucleotide

In one embodiment, the present invention relates to a single-stranded polynucleotide comprising a palindromic structure comprising an acyclic polynucleotide structural unit, wherein adenine in the palindromic structure is replaced by diaminopurine, and thymine at a position complementary to the adenine is replaced by a thiouracil derivative (which may be referred to as "the single-stranded polynucleotide of the present invention" in the present specification). This is explained below.

The acyclic polynucleotide structural unit is a structural unit corresponding to the nucleotide constituting the polynucleotide, and is not particularly limited as long as it does not contain a sugar skeleton. Typical examples of the acyclic polynucleotide structural unit include a structural unit represented by formula (1): 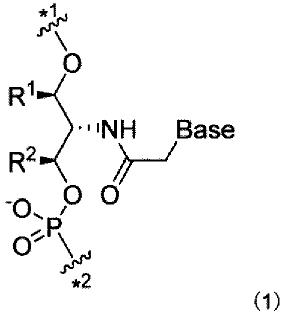 wherein R¹ and R² are the same or different, and each is a hydrogen atom or an organic group, excluding a case where R¹ and R² are both organic groups, and Base is a nucleobase.

The organic group is not particularly limited, and examples include hydrocarbon groups.

Preferred examples of hydrocarbon groups include chain hydrocarbon groups. Examples of chain hydrocarbon groups include alkyl, alkenyl, and alkynyl groups. Of these, alkyl groups are preferable. Specific examples of alkyl groups include methyl, ethyl, n-propyl, isopropyl, n-butyl, isobutyl, tert-butyl, sec-butyl, n-pentyl, neopentyl, n-hexyl, and 3-methylpentyl groups. The number of carbon atoms in the hydrocarbon group is not particularly limited. The number of carbon atoms is preferably 1 to 8, more preferably 1 to 6, even more preferably 1 to 4, still even more preferably 1 or 2, and particularly preferably 1. Further preferred is an alkyl group containing an alkynyl group (-C≡C-, -C≡CH) at the end or inside, which enables the introduction of various functional groups by click reactions etc.

In addition to the above examples, the organic group may be a monovalent group obtained by removing one hydrogen atom or functional group from various molecules, such as molecules used to modify polynucleotides. Examples of such molecules include polyethylene glycol chains, dye molecules, polycation (spermine), groove binders, amino groups, hydroxyl groups, thiol groups, metal ligands, photocleavable functional groups, sugar chains, and the like. These can be linked directly or indirectly to the skeleton of the above structural unit. For example, click reactions (e.g., the reaction of alkyne and azide described above) can be used for linkage.

Molecular modeling of acyclic polynucleotides, such as SNA and LaTNA, suggests that adopting relatively large organic groups as R¹ and R² would not affect the duplex formation ability or their structures.

In a preferred embodiment of the present invention, in terms of binding properties to target polynucleotides, such as miRNA, it is preferable that R¹ is a hydrogen atom or a chain hydrocarbon group, and that R² is a hydrogen atom.

As the nucleobase, bases constituting nucleic acids can be employed without restriction. Bases constituting nucleic acids include not only typical bases of natural nucleic acids such as RNA and DNA (adenine (A), thymine (T), uracil (U), guanine (G), cytosine (C), etc.), but also other bases such as hypoxanthine (I) and modified bases. Example of modified bases include pseudouracil, 3-methyluracil, dihydrouracil, 5-alkylcytosine (e.g., 5-methylcytosine), 5-alkyluracil (e.g., 5-ethyluracil), 5-halouracil (5-bromouracil), 6-azapyrimidine, 6-alkylpyrimidine (6-methyluracil), 4-acetylcytosine, 5-(carboxyhydroxymethyl)uracil, 5'-carboxymethylaminomethyl-2-thiouracil, 5-carboxymethylaminomethyluracil, 1-methyladenine, 1-methylhypoxanthine, 2,2-dimethylguanine, 3-methylcytosine, 2-methyladenine, 2-methylguanine, N6-methyladenine, 7-methylguanine, 5-methoxyaminomethyl-2-thiouracil, 5-methylaminomethyluracil, 5-methylcarbonylmethyluracil, 5-methyloxyuracil, 5-methyl-2-thiouracil, 2-methylthio-N6-isopentenyladenine, uracil-5-oxyacetic acid, 2-thiocytosine, purine, 2-aminopurine, isoguanine, indole, imidazole, xanthine, and the like.

In formula (1), *1 and *2 indicate the directions in which a polynucleotide is constituted. When R¹ is an organic group and R² is a hydrogen atom, *1 is the 3'-side and *2 is the 1'-side. When R¹ is a hydrogen atom and R² is an organic group, *1 is the 1'-side and *2 is the 3'-side. When R¹ is a hydrogen atom and R² is a hydrogen atom, *1 is the (S)-side and *2 is the (R)-side.

The palindromic structure is not particularly limited as long as it is a single-stranded polynucleotide consisting of a base sequence that can form a self-duplex (in the same molecule (hairpin) or between molecules (dimer)).

The palindromic structure comprises the acyclic polynucleotide structural unit. The ratio of the acyclic polynucleotide structural unit to 100% of the polynucleotide structural unit (the number of nucleotides) constituting the palindromic structure is, for example, 70% or more, preferably 80% or more, more preferably 90% or more, even more preferably 95% or more, and particularly preferably 100% (i.e., the palindromic structure consists of the acyclic polynucleotide structural unit). The ratio of the acyclic polynucleotide structural unit to 100% of the polynucleotide structural unit (the number of nucleotides) constituting the single-stranded polynucleotide of the present invention is, for example, 70% or more, preferably 80% or more, more preferably 90% or more, even more preferably 95% or more, and particularly preferably 100% (i.e., the single-stranded polynucleotide of the present invention consists of the acyclic polynucleotide structural unit). When at least part of the above ratio is satisfied, the problem of self-duplex formation becomes more prominent; thus, the effect of applying the technique of the present invention becomes more prominent. By satisfying at least part of the above ratio, resistance to enzymatic degradation in vivo can be further enhanced.

The palindromic structure is typically a single-stranded polynucleotide consisting of a base sequence represented by formula (2): A¹-B-A².

A¹ and A² are base sequences complementary to each other. That is, when A¹ is a base sequence: *1-ATGCCG, A² is a base sequence complementary to that base sequence, typically a base sequence: *1-CGGCAT (a completely complementary base sequence). In the present specification, the term "complementary" includes not only complete base complementarity (completely complementary: e.g., A and T or U, and G and C), but also complementarity to the extent that they can hybridize under stringent conditions. The stringent conditions can be determined based on the melting temperature (Tm) of nucleic acids, as taught by Berger and Kimmel (1987, Guide to Molecular Cloning Techniques Methods in Enzymology, Vol. 152, Academic Press, San Diego CA). Examples of washing conditions after hybridization generally include about "1×SSC, 0.1% SDS, 37°C." It is preferable that the hybridized state is maintained even after washing under such conditions. Although it is not particularly limited, examples of the washing conditions include more stringent hybridization conditions of about "0.5×SSC, 0.1% SDS, 42°C," and even more stringent hybridization conditions of about "0.1×SSC, 0.1% SDS, 65°C." Specifically, A² is a base sequence having an identity of, for example, 85% or more, preferably 90% or more, more preferably 95% or more, even more preferably 98% or more, still even more preferably 99% or more, and particularly preferably 100%, to the base sequence completely complementary to A¹.

The base length of A¹ and A² is preferably 3 or more. The base length is preferably 4 or more, 5 or more, 6 or more, 7 or more, 8 or more, 9 or more, or 10 or more. The upper limit of the base length is not particularly limited, and is, for example, 100, 50, 20, or 10. The base length of B is preferably an integer of 0 to (base length of A¹ + base length of A²)/4. The base length of the palindromic structure is preferably 6 or more, and more preferably 8 or more, 10 or more, 12 or more, 14 or more, 16 or more, 18 or more, or 20 or more. The upper limit of the base length of the palindromic structure is not particularly limited, and is, for example, 200, 100, 50, or 20. When at least part of these ranges are satisfied, the problem of self-duplex formation becomes more prominent; thus, the effect of applying the technique of the present invention becomes more prominent.

The ratio of the palindromic structure in the single-stranded polynucleotide of the present invention (the ratio of the polynucleotide structural unit constituting the palindromic structure to 100% of the polynucleotide structural unit (the number of nucleotides) constituting the single-stranded polynucleotide of the present invention) is not particularly limited as long as a self-duplex can be formed. The ratio is, for example, 10% or more, preferably 20% or more, more preferably 30% or more, even more preferably 40% or more, and still even more preferably 50% or more. When these ranges are satisfied, the problem of self-duplex formation becomes more prominent; thus, the effect of applying the technique of the present invention becomes more prominent.

In the single-stranded polynucleotide of the present invention, structural units other than the acyclic polynucleotide structural unit are not particularly limited, and structural units of natural nucleic acids and various artificial nucleic acids, including aTNA and SNA, can be employed. In addition to DNA, RNA, etc., the nucleic acids that can be employed may be specifically those that have undergone known chemical modifications, as shown below. In order to prevent degradation by hydrolytic enzymes such as nucleases, phosphate residues (phosphates) of each nucleotide can be replaced by chemically modified phosphate residues, such as phosphorothioate (PS), methylphosphonate, and phosphorodithionate. Alternatively, the hydroxyl group at the 2'-position of the sugar (ribose) of each ribonucleotide may be replaced by -OR (R is, for example, CH3 (2'-O-Me), CH2CH2OCH3 (2'-O-MOE), CH2CH2NHC(NH)NH2, CH2CONHCH3, CH2CH2CN, or the like). Further, the base moiety (pyrimidine or purine) may be chemically modified; for example, a methyl group or a cationic functional group is introduced at the 5-position of the pyrimidine base, or the carbonyl group at the 2-position is replaced by thiocarbonyl. Other examples include, but are not limited to, modification of the phosphate moiety or hydroxyl moiety with biotin, an amino group, a lower alkyl amine group, an acetyl group, or the like. In addition, for example, BNA (LNA) in which the conformation of the sugar moiety is fixed to N-type by crosslinking 2'-oxygen and 4'-carbon in the sugar moiety of the nucleotide can also be preferably used.

In the single-stranded polynucleotide of the present invention, adenine and/or an analog thereof in the palindromic structure (not containing diaminopurine and a thiouracil derivative) is replaced by diaminopurine (2,6-diaminopurine), and thymine and/or an analog thereof at a position complementary to the adenine is replaced by a thiouracil derivative (2-thiouracil, 2-thiothymine, etc.). Complementary positions are opposite positions when palindromic structures are arranged so as to be complementary to each other. For example, when the base sequence of the palindromic structure is a base sequence: *1-TAACGTTA, the complementary position of the second A from the *1-side is the position of the seventh T from the *1-side.

It is patricianly preferable that the number of diaminopurines and the number of thiouracil derivatives are each 2 or more. This can further enhance the binding properties of a single-stranded polynucleotide comprising a palindromic structure comprising an acyclic polynucleotide structural unit to target polynucleotides, such as miRNA, while suppressing self-duplex formation of the single-stranded polynucleotide. The upper limit of this number is not particularly limited, and is, for example, 50, 20, 10, or 5. Further, diaminopurines and thiouracil derivatives are preferably present individually and/or adjacent to each other.

The ratio of the sum of diaminopurines and thiouracil derivatives to 100% of the number of bases in the palindromic structure is, for example, 10 to 90%, preferably 20 to 80%, more preferably 30 to 70%, and even more preferably 40 to 60%.

In the single-stranded polynucleotide of the present invention, it is preferable that thymine and/or an analog thereof in structures other than the palindromic structure is not replaced by a thiouracil derivative.

The base length of the single-stranded polynucleotide of the present invention is not particularly limited, and is, for example, 6 to 10000. The base length is preferably 6 to 1000, more preferably 6 to 500, even more preferably 6 to 200, still even more preferably 6 to 100, and particularly preferably 6 to 50, in terms of ease of synthesis.

The single-stranded polynucleotide of the present invention also includes those in which other molecules are linked together. Other molecules are not particularly limited, and examples include fluorescently labeled substances, biotin, azide, ethynyl groups, and the like. Examples of fluorescently labeled substances include fluorescein, rhodamine, Texas Red, tetramethylrhodamine, carboxyrhodamine, phycoerythrin, 6-FAM^{™}, Cy^{™}3, Cy^{™}5, Alexa Fluor^{™} series, and the like.

The single-stranded polynucleotide of the present invention can bind to a target polynucleotide to exhibit various functions. Therefore, the single-stranded polynucleotide of the present invention (preferably palindromic structure) is complementary to a part or the whole of the target polynucleotide. Examples of the target polynucleotide include miRNA, mRNA, DNA, and the like. The target polynucleotide is preferably a cellular endogenous polynucleotide. The single-stranded polynucleotide of the present invention can be preferably used as an anti-miRNA polynucleotide.

Further, in one embodiment, the present invention can also provide a double-stranded polynucleotide comprising the single-stranded polynucleotide of the present invention.

The single-stranded polynucleotide of the present invention can be easily produced according to a known genetic engineering method, a method for producing artificial nucleic acids, or the like. For example, PCR, restriction endonuclease cleavage, and nucleic acid linkage technology can be used for production. Preferably, the single-stranded polynucleotide of the present invention can be produced according to the production method of the present invention, described later.

The single-stranded polynucleotide of the present invention and the double-stranded polynucleotide (which may be collectively referred to as "the polynucleotide of the present invention") can be used for pharmaceuticals, reagents, and the like (hereinafter, which may be collectively referred to as "the agent of the present invention").

The agent of the present invention is not particularly limited as long as it comprises the polynucleotide of the present invention, and other components may be further contained, if necessary. Other components are not particularly limited as long as they are pharmaceutically acceptable components. Examples include bases, carriers, solvents, dispersants, emulsifiers, buffers, stabilizers, excipients, binders, disintegrants, lubricants, thickeners, moisturizers, colorants, fragrances, chelating agents, and the like.

The usage of the agent of the present invention is not particularly limited, and a suitable usage can be adopted depending on the type of agent. The agent of the present invention can be used, for example, in vitro (e.g., added to the medium of cultured cells) or in vivo (e.g., administered to an animal).

The target to which the agent of the present invention is applied is not particularly limited. Examples include various mammalian animals, such as humans, monkeys, mice, rats, dogs, cats, and rabbits; animal cells; and the like. The type of cell is also not particularly limited, and examples include blood cells, hematopoietic stem cells and precursor cells, gametes (sperm and ovum), fibroblasts, epithelial cells, vascular endothelial cells, nerve cells, liver cells, keratin-producing cells, muscle cells, epidermal cells, endocrine cells, ES cells, iPS cells, tissue stem cells, cancer cells, and the like.

When the agent of the present invention is used as an anticancer drug, the type of cancer cell is not particularly limited. Examples include kidney cancer cells, leukemia cells, esophageal cancer cells, gastric cancer cells, colon cancer cells, liver cancer cells, pancreatic cancer cells, lung cancer cells, prostate cancer cells, skin cancer cells, breast cancer cells, cervical cancer cells, and the like.

The agent of the present invention can have any dosage form, and examples include oral dosage forms, such as tablets (including orally disintegrating tablets, chewable tablets, foaming tablets, troches, jelly drops, etc.), pills, granules, fine granules, powders, hard capsules, soft capsules, dry syrups, liquids (including drinks, suspensions, and syrups), and jellies; and parenteral dosage forms, such as injectable preparations (e.g., drip infusion (e.g., intravenous drip infusion), intravenous injection, intramuscular injection, subcutaneous injection, and intradermal injection), external preparations (e.g., ointments, poultices, and lotions), suppositories, inhalers, eye drops, eye ointments, nasal drops, ear drops, and liposomes.

The route of administration of the agent of the present invention is not particularly limited as long as desired effects are obtained. Examples include intestinal administration, such as oral administration, tube nutrition, and enema administration; parenteral administration, such as intravenous administration, intraarterial administration, intramuscular administration, intracardiac administration, subcutaneous administration, intradermal administration, and intraperitoneal administration; and the like. The content of the polynucleotide of the present invention in the agent of the present invention depends on the usage, the application target, the conditions of the application target, and the like, and is not limited. For example, the content is 0.0001 to 95 wt.%, and preferably 0.001 to 50 wt.%.

The dosage amount of the agent of the present invention when administered to an animal is not particularly limited as long as it is an amount effective to exhibit drug efficacy. In general, the weight of the polynucleotide of the present invention as an active ingredient when orally administered is 0.1 to 1000 mg/kg body weight per day, and preferably 0.5 to 50 mg/kg body weight per day, and when parenterally administered, is 0.01 to 100 mg/kg body weight, and preferably 0.1 to 10 mg/kg body weight. The above dosage amount is preferably administered once a day, or two or three times a day, and can be appropriately increased or decreased depending on the age, pathological condition, and symptom.

### 2. Amidite Monomer

In one embodiment, the present invention relates to a compound or a salt thereof or a solvate thereof (which may be referred to as "the amidite monomer of the present invention" in the present specification), the compound being represented by formula (3): 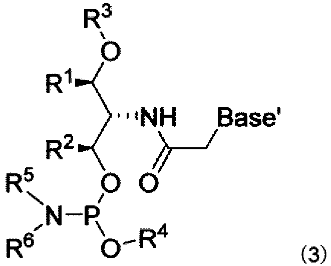 wherein R¹ and R² are the same or different, and each is a hydrogen atom or an organic group, excluding a case where R¹ and R² are both organic groups, R³ and R⁴ are the same or different, and each is a protecting group for a hydroxyl group, R⁵ and R⁶ are the same or different, and each is an alkyl group, and Base' is a nucleobase in which an amino group of diaminopurine is protected by a protecting group removable by an acid, or a nucleobase in which a thiocarbonyl group of a thiouracil derivative is protected by a protecting group removable by an acid. This is explained below.

R¹, R², diaminopurine, and the thiouracil derivative are as explained in the "1. Single-Stranded Polynucleotide" section above.

As R³, any group that can function as a protecting group for a hydroxyl group can be used without any restriction, and a wide range of known protecting groups used for amidite monomers can be used. R³ is preferably, for example, a group represented by formula (4): 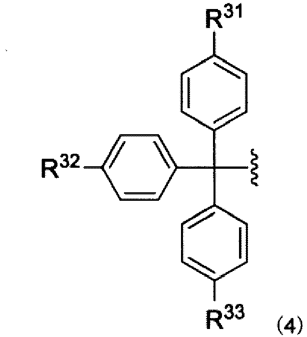 wherein R³¹, R³², and R³³ are the same or different, and each is a hydrogen atom or an alkoxy group.

It is preferable that one of R³¹, R³², and R³³ is hydrogen, while the other two moieties are alkoxy groups. Particularly preferred alkoxy groups are methoxy groups.

As R⁴, any group that can function as a protecting group for a hydroxyl group can be used without any restriction, and a wide range of known protecting groups used for amidite monomers can be used. Examples of R⁴ include alkyl, alkenyl, alkynyl, cycloalkyl, haloalkyl, aryl, heteroaryl, arylalkyl, cycloalkenyl, cycloalkylalkyl, cyclic alkyl, hydroxyalkyl, aminoalkyl, alkoxyalkyl, heterocyclic alkenyl, heterocyclic alkyl, heteroarylalkyl, silyl, silyloxyalkyl, mono-, di-, or trialkylsilyl, and mono-, di-, or trialkylsilyloxyalkyl groups. These may be substituted with electron-withdrawing groups.

R⁴ is preferably an alkyl group substituted with an electron-withdrawing group. Examples of the electron-withdrawing group include cyano, nitro, alkylsulfonyl, halogen, arylsulfonyl, trihalomethyl, and trialkylamino groups; a cyano group is preferred. R⁴ is particularly preferably -(CH₂)₂-CN.

The alkyl groups represented by R⁵ and R⁶ may be linear or branched, and are preferably C₁₋₁₂ alkyl groups, and more preferably C₁₋₆ alkyl groups. Examples of alkyl groups include methyl, ethyl, n-propyl, isopropyl, n-butyl, isobutyl, tert-butyl, n-pentyl, isopentyl, and hexyl. The alkyl groups as mentioned herein include the alkyl moiety of an alkoxy group etc. R⁵ and R⁶ may be bonded together to form a ring structure.

R⁵ and R⁶ are particularly preferably both isopropyl groups.

The protecting group removable by an acid is not particularly limited, and examples include a Boc group (tert-butoxycarbonyl group), an MMPM group (4-methoxy-2-methylbenzyl group), and the like.

A preferred embodiment of the compound represented by formula (3) is a compound represented by formula (3A) or (3B): 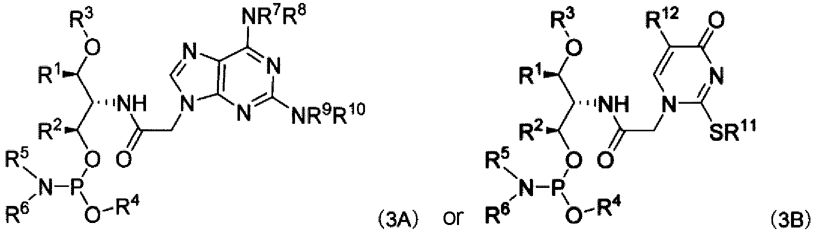 wherein R¹, R²/R³, R⁴, R⁵, and R⁶ are as defined above, R⁷, R⁸, R⁹, R¹⁰, and R¹¹ are the same or different, and each is a protecting group removable by an acid, and R¹² is a hydrogen atom or an alkyl group (preferably a methyl group).

The salt of the amidite monomer of the present invention is not particularly limited, and examples include salts with inorganic bases, such as sodium salt, magnesium salt, potassium salt, calcium salt, and aluminum salt; salts with organic bases, such as methylamine, ethylamine, and ethanolamine; salts with basic amino acids, such as lysine, ornithine, and arginine; and ammonium salts. These salts may be acid addition salts. Specific examples of such salts include acid addition salts with mineral acids, such as hydrochloric acid, hydrobromic acid, hydroiodic acid, sulfuric acid, nitric acid, and phosphoric acid; acid addition salts with organic acids, such as formic acid, acetic acid, propionic acid, oxalic acid, malonic acid, malic acid, tartaric acid, fumaric acid, succinic acid, lactic acid, maleic acid, citric acid, methanesulfonic acid, trifluoromethanesulfonic acid, and ethanesulfonic acid; and acid addition salts with acidic amino acids, such as aspartic acid and glutamic acid. The amidite monomer of the present invention includes hydrates, solvates, crystalline polymorphs, and the like.

The amidite monomer of the present invention can be produced by the methods described in the Examples below or by methods with appropriate modifications to these methods, as necessary.

### 3. Method for Producing Single-Stranded Polynucleotide

In one embodiment, the present invention relates to a method for producing a single-stranded polynucleotide by a phosphoramidite method using the amidite monomer of the present invention (which may be referred to as "the production method of the present invention" in the present specification). This is explained below.

The phosphoramidite method can be performed by a known method using, for example, a commercially available nucleic acid automatic synthesizer. Specifically, the phosphoramidite method comprises, for example, (A) a step of deprotecting the hydroxyl group at the 5'-position (or an equivalent position), (B) a step of condensing the amidite monomer of the present invention, (C) a step of capping the hydroxyl group at the 5'-position (or an equivalent position) of the unreacted compound, (D) a step of converting the phosphite group to a phosphate group or a thiophosphate group, (E) a step of cutting out the obtained compound from the solid-phase carrier and deprotecting the hydroxyl group at the 2'-position and a hydroxyl group in the nucleobase, and (F) a step of deprotecting the hydroxyl group at the 5'-position (or an equivalent position). A compound comprising a polynucleotide skeleton having a desired chain length can be produced by repeating steps (A) to (D).

In step (D), it is preferable to use tert-butyl hydroperoxide as an oxidizing agent.

In the deprotection stage in step (E), generally, the protecting group of a nucleobase derived from the amidite monomer of the present invention is first removed by an acid such as TFA, and then deprotection is performed with a base such as ammonia water. The time for deprotection with an acid such as TFA is preferably 2 to 6 hours. In deprotection with a base, a longer treatment time causes the breakage of the thiouracil derivative; thus, in order to prevent this, the treatment time is preferably about 4 to 6 hours.

The obtained single-stranded polynucleotide can be isolated and purified, if necessary. In general, isolation can be achieved by precipitation, extraction, and purification of RNA. Specifically, RNA is precipitated by adding a solvent with low solubility for RNA, such as ethanol or isopropyl alcohol, to the solution after the reaction, or a solution of phenol/chloroform/isoamyl alcohol is added to the reaction solution, and RNA is extracted into the aqueous layer. Then, isolation and purification can be achieved by known high-performance liquid chromatography (HPLC) techniques, such as reverse-phase column chromatography, anion-exchange column chromatography, and affinity column chromatography.

The single-stranded polynucleotide of the present invention can be preferably produced by the production method of the present invention.

### Examples

The present invention is described in detail below based on Examples; however, the present invention is not limited to these Examples.

### Synthesis Example 1: Synthesis of Boc-Protected 2,6-Diaminopurine SNA

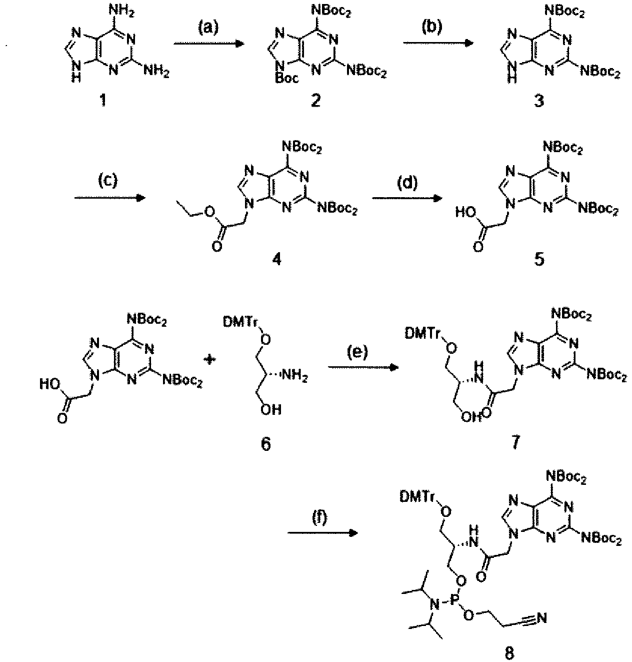

The reagents and conditions are as follows. a) Boc₂O, DMAP, THF, rt, 18 h; b) NaHCO₃, MeOH-H₂O (2:1), 50°C, 1 h, then rt, 18 h; c) BrCH₂CO₂Et, K₂CO₃, DMF, rt, 20 h; d) NaOH, MeOH, 1,4-dioxane, H₂O, 0°C, 10 min; e) DMT-MM, DMF, triethylamine, r.t., 1 h; f) 2-cyanoethyl N,N-diisopropylchlorophosphoramidite, dry CH₂Cl₂, Et₂N, 0°C, 30 min.

### Synthesis Example 1-1: Synthesis of Compound 2 (Step a)

2,6-Diaminopurine (compound 1) (5.0 g, 33.3 mmol) and DMAP (0.61 g, 5.0 mmol) were dissolved in THF (100 to 200 mL) in a nitrogen atmosphere. Boc₂O (61.2 g, 64.4 mL) was added to this solution at 0°C. After stirring at 0°C for 5 minutes, the mixture was stirred at room temperature for 18 hours. After completion of the reaction, the solvent was removed by evaporation. The residue was dissolved in CHCl₃, and then mixed with H₂O. The organic layer was collected and dried over MgSO₄, and the solvent was removed by evaporation. The residue was purified by silica gel column chromatography (hexane/AcOEt, 10:3), thereby obtaining compound 2 (yield: 82%).
¹H-NMR [CDCl₃, 500 MHz] δ=8.53 (s, 1H, 8-purine), 1.69 (s, 9H), 1.45 (s, 18H), 1.43 (s, 18H)
¹³C-NMR [CDCl₃, 125 MHz] δ=153.7, 152.9, 151.6, 150.6, 149.7, 145.5, 143.8, 127.8, 87.3, 83.9, 83.2, 41.9, 27.8, 27.7, 27.6
HRMS (FAB) Calcd for compound 2 (M+H+) 651.3348. Found 651.3371.

### Synthesis Example 1-2: Synthesis of Compound 3 (Step b)

Compound 2 (17.6 g, 27 mmol) was dissolved in AcOEt (400 mL), and washed with 1 M HCl (30 mL) and a saturated solution of NaCl. The organic layer was dried over MgSO₄, and the solvent was removed by evaporation, followed by vacuum drying. The mixture was dissolved in MeOH (200 mL), a NaHCO₃ solution (90 mL) was added, and the mixture was placed in a 50°C oil bath and stirred for 1 hour. After completion of the reaction, MeOH was removed by evaporation, and H₂O (100 mL) was added. The resultant was extracted with CHCl₃ (50 mL) three times, the organic layer was combined and dried over MgSO₄, and the solvent was removed by evaporation. The residue was purified by silica gel column chromatography (hexane/AcOEt, from 2:1 to 100% of AcOEt), thereby obtaining 14.3 g of compound 3 (yield: 96%).
¹H-NMR [CDCl₃, 500 MHz] δ=8.38 (s, 1H), 1.49 (s, 18H), 1.44 (s, 18H)
¹³C-NMR [CDCl₃, 125 MHz] δ=151.66, 150.95, 150.06, 144.99, 83.58, 27.99
HRMS (FAB) Calcd for compound 3 (M+H+) 551.2824. Found 551.2829.

### Synthesis Example 1-3: Synthesis of Compound 4 (Step c)

Compound 3 (14.3 g, 26.0 mmol) and K₂CO₃ (1.15 equivalents, 4.0 g, 29.9 mmol) were suspended in dry DMF (60 mL) in a nitrogen atmosphere. Ethyl bromoacetate (1.1 equivalents, 28.6 mmol) was added dropwise to the mixture at 0°C, and then stirred for 10 minutes. Next, the mixture was stirred at room temperature for 20 hours. After the reaction solution was removed by toluene azeotrope, H₂O (150 mL) was added. The resultant was extracted with CHCl₃ (100 mL) three times, and the organic layer was combined and washed with H₂O. The organic layer was dried over MgSO₄, and the solvent was removed by evaporation. The residue was purified by silica gel column chromatography (CHCl₃/MeOH, 40:1), thereby obtaining 14.6 g of compound 4 (yield: 88%).
¹H-NMR [CDCl₃, 500 MHz] δ=8.18 (s, 1H), 5.03 (s, 2H), 4.27-4.26 (d, 2H), 1.42 (s, 18H), 1.41 (s, 18H), 1.30 (m, 3H)
¹³C-NMR [CDCl₃, 125 MHz] δ=166.69, 154.38, 152.35, 151.18, 150.85, 145.92, 83.84, 83.36, 62.49, 44.47, 27.88, 27.75, 14.16
HRMS (FAB) Calcd for compound 4 (M+H+) 637.3192 Found 637.3197.

### Synthesis Example 1-4: Synthesis of Compound 5 (Step d)

Compound 4 (14.6 g, 23 mmol) was dissolved in MeOH (120 mL) and 1,4-dioxane (40 mL). Next, a 2 M NaOH solution (40 mL) was added on ice, and stirred for 10 minutes. After completion of the reaction, 2 M HCl was added to adjust the pH of the reaction solution to 3, and MeOH and 1,4-dioxane were removed by evaporation. The resultant was extracted with AcOEt three times, and the organic layer was combined and washed with H₂O. The organic layer was dried over MgSO₄, and the solvent was removed by evaporation, followed by vacuum drying. The crude product (compound 5) was used in the next reaction step without further purification (quantitative yield).
¹H-NMR [CDCl₃, 500 MHz] δ=8.46 (s, 1H), 5.03 (s, 2H), 1.41 (s, 18H), 1.40 (s, 18H)
¹³C-NMR [CDCl₃, 125 MHz] δ=168.9, 154.31, 152.28, 150.94, 150.59, 149.97, 147.2, 126.28, 84.06, 83.61, 27.85, 27.74
HRMS (FAB) Calcd for compound 5 (M+H+) 609.2879. Found 609.2900.

### Synthesis Example 1-5: Synthesis of Compound 7 (Step e)

Compound 5 (5.4 g, 8.8 mmol) was dissolved in DMF, and condensed with 1.96 g (1.0 equivalent, 8.0 mmol) of compound 6 in the presence of Et₃N (3.0 equivalents) and DMT-MM(1.2 equivalents, 5 mmol). After completion of the reaction, CHCl₃ was added, and the organic layer was washed with saturated NaHCO₃. The mixture was dried over MgSO₄. The solvent was removed by evaporation, and the residue was purified by silica gel column chromatography (CHCl₃/MeOH, from 30:1 to 10:1, containing 3% Et₃N), thereby obtaining 7.44 g (7.6 mmol) of compound 7 (yield: 94%).
¹H-NMR [CDCl₃, 500 MHz] δ=8.19 (s, 1H), 7.45-7.20 (m, 9H), 6.88-6.80 (m, 4H), 6.59-6.55 (d, 1H), 4.91-4.75 (dd, 2H), 4.18-4.10 (m, 1H), 3.85-3.80 (m, 1H), 3.79 (s, 6H), 3.70-3.65 (m, 1H), 3.35- 3.20 (m, 2H), 1.40 (s, 18H), 1.39 (s, 18H)
¹³C-NMR [CDCl₃, 125 MHz] δ=165.28, 162.69, 158.69, 154.38, 152.11, 151.13, 151.09, 146.44, 135.79, 130.08, 130.03, 128.12, 128.05, 127.07, 113.35, 86.58, 83.98, 83.67, 62.53, 62.41, 55.31, 51.55, 46.16, 36.58, 31.55; 28.31, 28.21, 27.91, 27.77
HRMS (FAB) Calcd for compound 7 (M+H+) 984.4713. Found 984.4686.

### Synthesis Example 1-6: Synthesis of Compound 8 (Step f)

Compound 7 (6.46 g, 6.56 mmol) was dissolved in dehydrated CH₂Cl₂ (7 mL) and Et₃N (4.55 mL), and 2.2 mL (7.84 mmol) of 2-cyanoethyl N,N-diisopropylchlorophosphoramidite was added dropwise in a nitrogen atmosphere at 0°C. After stirring at room temperature for 20 minutes, the reaction mixture was purified by silica gel column chromatography (hexane/AcOEt, from 4:1 to 1:1, containing 3% Et₃N), thereby obtaining 4.81 g (4.06 mmol) of compound 8 (yield: 62%).
¹H-NMR [CDCl₃, 500 MHz] δ=8.23-8.19 (1H), 7.43-7.20 (m, 9H), 6.85-6.82 (m, 4H), 6.37, (m, 1H), 4.93-4.73 (m, 2H), 4.40-4.20 (m, 1H), 4.00-3.80 (m, 2H), 3.79(s, 6H), 3.70-3.60 (m, 1H), 3.61-3.52 (m, 2H), 3.42-3.35 (m, 1H), 3.21-3.15 (m, 1H), 2.70-2.40 (m, 2H), 1.41 (s, 18H), 1.39 (s, 18H), 1.20-1.10 (m, 12H)
¹³C-NMR [CDCl₃, 125 MHz] δ=164.77, 158.58, 154.41, 152.05, 150.84, 150.03, 146.58, 146.48, 144.73, 144.63, 135.91, 135.83, 135.75, 130.13, 130.09, 130.07, 130.04, 128.16, 128.11, 127.93, 126.94, 126.80, 118.59, 118.48, 113.23, 113.20, 86.29, 86.27, 83.73, 83.72, 83.28, 83.24, 65.86, 61.45, 58.54, 58.44, 58.40, 58.29, 55.26, 50.43, 50.37, 45.55, 45.46, 43.18, 43.08, 30.95, 27.82, 27.71, 24.68, 24.62, 20.62, 20.60, 20.60, 20.56, 15.31
³¹P-NMR [CDCl₃, 202 MHz] δ=148.15, 147.71
HRMS (FAB) Calcd for compound 8 (M+H+) 1185.5792. Found 1184.5794.

### Synthesis Example 2: Synthesis of MMPM-Protected Thiouracil SNA

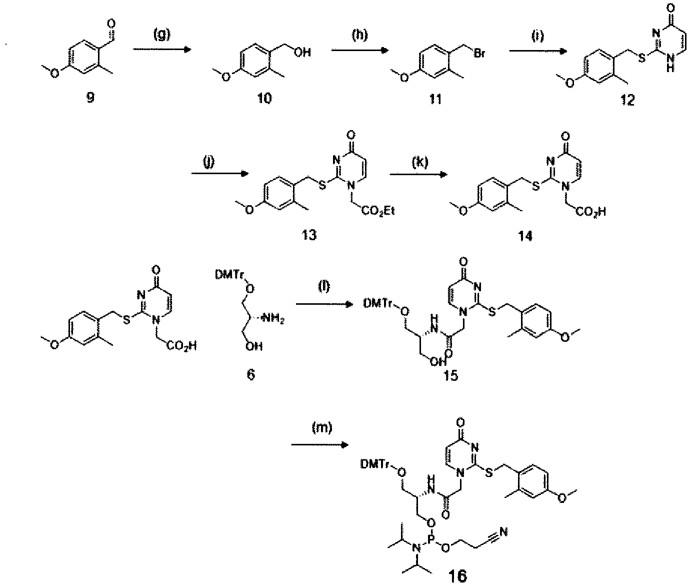

The reagents and conditions are as follows. g) NaBH₄, EtOH, r.t., 5 min; h) PBr₃, CH₂Cl₂, 0°C, 2.5 h; i) 2-thiouracil, KOH, EtOH, H₂O, r.t., overnight; j) BrCH₂CO₂Et, NaOEt, EtOH, reflux, 2 h; k) LiOH, THF, MeOH, H₂O, r.t., overnight; 1) DMT-MM, DMF, triethylamine, r.t., 1 h; m) 2-cyanoethyl N,N-diisopropylchlorophosphoramidite, dry CH₂Cl₂, triethylamine, 0°C, 30 min.

### Synthesis Example 2-1: Synthesis of Compound 10 (Step g)

Compound 9 (2.03 g, 12.8 mmol) was dissolved in EtOH (32 mL), and then NaBH₄ was added. The mixture was stirred at room temperature for 5 minutes. After completion of the reaction, H₂O (240 mL) was mixed, and 4 M HCl was added to adjust the pH of the reaction solution to 4. The resultant was extracted with Et₂O three times, and the organic layer was combined and dried over MgSO₄. The solvent was removed by evaporation, and the obtained crude product (compound 10) was used in the next step without further purification (quantitative yield).
¹H-NMR [CDCl₃, 500 MHz] δ=7.22 (d, 1H), 6.74-6.71 (m, 2H), 4.61 (s, 2H), 3.79 (s, 3H), 2.36 (s, 3H), 1.63 (s, 1H)
¹³C-NMR [CDCl₃, 125 MHz] δ=158.82, 137.70, 131.14, 129.17, 115.91, 129.17, 115.91, 110.47, 62.41, 54.94, 18.66
HRMS (FAB) Calcd for compound 10 (M+H⁺) 152.0837. Found 152.0837.

### Synthesis Example 2-2: Synthesis of Compound 12 (Steps h and i)

Compound 10 (1.93 g, 12.7 mmol) was dissolved in dehydrated CH₂Cl₂ in a nitrogen atmosphere. PBr₃ was added dropwise at 0°C over 2.5 hours and stirred for 15 minutes, and then NaHCO₃ mixed with crushed ice was mixed with the reaction solution. The resultant was extracted with Et₂O three times, the organic layer was combined and dried over MgSO₄, and the solvent was removed by evaporation. The obtained crude product (compound 11) was used in the next step without further purification (quantitative yield).

2-Thiouracil (833 mg, 6.50 mmol) was suspended in EtOH (8 mL), and a 1 M KOH solution (8 mL) was added, followed by heating at 45°C. After cooling to room temperature, compound 11 dissolved in EtOH (5 mL) was added dropwise and stirred at room temperature overnight. The reaction solution was mixed with a saturated NaHCO₃ solution (15 mL). The precipitate was collected by filtration, washed with H₂O, EtOH, AcOEt, and Et₂O, and then vacuum-dried, thereby obtaining compound 12 (yield: 72%).
¹H-NMR [CDCl₃, 500 MHz] δ=7.91 (s, 1H), 7.29-7.28 (d, 1H), 6.78-6.78 (d, 1H), 6.72-6.70 (dd, 1H), 6.12 (s, 1H), 4.35 (s, 2H), 3.71 (s, 3H), 2.31 (s, 3H)
¹³C-NMR [CDCl₃, 125 MHz] δ=163.98, 143.39, 136.45, 131.13, 121.05, 116.53, 37.10, 24.57
HRMS (FAB) Calcd for compound 12 (M+H⁺) 263.0849. Found 263.0844.

### Synthesis Example 2-3: Synthesis of Compound 13 (Step j)

Compound 12 (1.74 g, 4.71 mmol) was dissolved in dry EtOH and then in NaOEt (2.0 equivalents, 20% in EtOH). The solution was refluxed at 90°C, mixed with BrCH₂CO₂Et (2.0 equivalents), and stirred for 2 hours. After cooling to room temperature, a saturated NaHCO₃ solution (30 mL) was mixed, and EtOH was removed by evaporation. The resultant was extracted with a mixed solution of CH₂Cl₂ and MeOH (3:1) three times. The organic layer was combined and dried over MgSO₄, followed by evaporation removal. The residue was purified by silica gel column chromatography (hexane/AcOEt, from 5:1 to 1:1, AcOEt, AcoEt/MeOH, 9:1), thereby obtaining 0.63 g (1.81 mmol) of compound 13 (yield: 38%) .
¹H-NMR [CDCl₃, 500 MHz] δ=7.26-7.24 (d, 1H), 6.73-6.71 (d, 1H), 6.70-6.67 (dd, 1H), 6.08-6.05 (d, 1H), 4.56 (s, 2H), 4.46 (s, 2H), 4.26-4.21 (q, 2H), 3.77 (s, 3H), 2.34 (s, 3H), 1.27-1.24 (t, 3H)
¹³C-NMR [CDCl₃, 125 MHz] δ=167.91, 166.04, 163.40, 159.54, 144.06, 138.89, 131.83, 124.28, 116.24, 111.44, 109.88, 62.63, 55.21, 52.83, 34.87, 19.51, 14.01
HRMS (FAB) Calcd for compound 13 (M+H⁺) 349.1217. Found 349.1233.

### Synthesis Example 2-4: Synthesis of Compound 14 (Step k)

Compound 13 (0.63 g, 1.81 mmol) was dissolved in MeOH (4.7 mL), THF (11.1 mL), and 2 M LiOH (1.6 mL), and stirred at room temperature for 10 minutes. The solvent was removed by evaporation, and the precipitate was filtered and washed with AcOEt and Et₂O, and vacuum-dried. The obtained crude product (compound 14) was used in the next step without further purification (quantitative yield).
¹H-NMR [CDCl₃, 500 MHz] δ=7.52-7.51 (d, 1H), 7.45-7.43 (d, 1H), 7.31-7.28 (d, 1H), 6.78-6.76 (d,1H), 6.72-6.68 (dd, 1H), 5.82-5.80 (d,1H), 5.72-5.69 (d, 1H), 4.28 (s, 1H), 4.10 (s, 1H), 4.05 (s, 1H), 3.79 (s 1H), 3.71 (s, 1H), 2.31 (s, 3H), 1.66 (s, 3H) ¹³C-NMR [CDCl₃, 125 MHz] δ=175.82, 170.59, 168.50, 167.28, 167.90, 162.26, 158.81, 156.59, 146.39, 145.05, 138.36, 131.43, 125.71, 115.82, 111.34, 107.70, 106.63, 55.78, 55.05, 54.78, 53.26, 32.85, 25.22, 19.13.
HRMS (FAB) Calcd for compound 14 (M+H⁺) 327.0985. Found 327.0988.

### Synthesis Example 2-5: Synthesis of Compound 15 (Step I)

Compound 14 (0.57 g, 1.67 mmol) was dissolved in DMF, and subjected to coupling with 0.64 g (1.6 equivalents, 2.59 mmol) of compound 6 in the presence of Et₃N (3.0 equivalents) and DMT-MM (1.8 equivalents). After the reaction, the mixture was diluted with CHCl₃, and the organic layer was washed with saturated NaHCO₃. The mixture was dried over MgSO₄. The solvent was removed by evaporation, and the residue was purified by silica gel column chromatography (CHCl₃/MeOH, from 20:1 to 10:1, containing 2% Et₃N), thereby obtaining 1.32 g (0.93 mmol) of compound 7 (yield: 56%).
¹H-NMR [CDCl₃, 500 MHz] δ=7.37-7.35 (m, 2H), 7.27-7.00 (m, 10H), 6.79-6.76 (m 4H), 6.66-6.64 (d, 1H), 6.58-6.55 (m, 1H), 5.98-5.96 (d, H), 4.47-4.40 (dd, 2H), 4.35-4.20 (dd, 2H), 4.14 (m, 1H), 3.78-3.73 (m, 10H), 3.69-3.65 (m, 1H), 3.29-3.19 (m, 2H), 2.20 (s, 3H).
¹³C-NMR [CDCl₃, 125 MHz] δ=168.53, 164.87, 163.95, 159.59, 158.69, 158.67, 144.76, 144.68, 138.97, 135.83, 135.72, 131.92, 130.14, 130.05, 128.12, 128.03, 127.04, 124.32, 116.35, 113.34, 111.51, 109.71, 86.51, 62.71, 55.34, 54.44, 52.05, 46.13, 34.99, 19.55. HRMS (FAB) Calcd for compound 15 (M+H⁺) 696.2738. Found 696.2768.

### Synthesis Example 2-6: Synthesis of Compound 16 (Step m)

Compound 15 (0.92 g, 1.32 mmol) was dissolved in dry CH₂Cl₂ (5 mL) and Et₃N (0.9 mL), and 0.44 mL (1.57 mmol) of 2-cyanoethyl N,N-diisopropylchlorophosphoramidite was added dropwise in a nitrogen atmosphere at 0°C. After stirring at room temperature for 20 minutes, the reaction solution was purified by silica gel column chromatography (CHCl₃/acetone, containing 3% Et₃N, from 4:1 to 2:1), thereby obtaining 0.78 g (0.87 mmol) of compound 8 (yield: 66%).
¹H-NMR [CDCl₃, 500 MHz] δ=7.40-7.38 (m, 2H), 7.30-7.18 (m, 9H), 7.11-7.07 (d, 1H), 6.81-6.78 (m, 4H), 6.69-6.66 (m, 1H), 6.65-6.59 (m, 1H),6.43-6.38 (m, 1H), 6.03-6.036 (m, 1H), 4.51-4.29 (m, 5H), 3.93-3.85 (m, 1H), 3.77 (m, 10H), 3.56-3.45 (m, 3H), 3.35-3.25 (m, 1H), 3.18-3.12 (m, 1H), 2.52-2.35 (m, 2H), 2.26-2.25 (m, 3H), 1.15-1.05 (m, 12H)
¹³C-NMR [CDCl₃, 125 MHz] δ=164.77, 158.58, 154.41, 152.05, 150.84, 150.03, 146.58, 146.48, 144.73, 144.63, 135.91, 135.83, 135.75, 130.13, 130.09, 130.07, 130.04, 128.16, 128.11, 127.93,126.94, 126.80, 118.59, 118.48, 113.23, 113.20, 86.29, 86.27, 83.73, 83.72, 83.28, 83.24, 65.86, 61.45, 58.54, 58.44, 58.40, 58.29, 50.43, 50.37, 45.55, 45.46, 43.18, 43.08, 30.95, 27.82, 27.71, 24.68, 24.62, 20.62, 20.60, 20.56, 15.31
³¹P-NMR [CDCl₃, 202 MHz] δ=148.03, 147.76
HRMS (FAB) Calcd for compound 16 (M+H⁺) 896.3817. Found 896.3824.

### Synthesis Example 3: Synthesis 1 of Single-Stranded

### Polynucleotides

Four single-stranded polynucleotides consisting of a palindromic structure (D represents 2,6-diaminopurine, and sU represents 2-thiouracil) shown below were prepared. SNAa, D2, and sU2 are SNA polynucleotides. RNAb is an RNA polynucleotide.
- SNAa: (S)-ATTGCAAT-(R)
- RNAb: 5'-AUUGCAAT-3'
- SNAa-D2: (S)-ATTGCDDT-(R)
- SNAa-sU2: (S)-AsUsUGCDDT-(R).

SNAa and RNAb were obtained from Fasmac and Hokkaido System Science Co., Ltd.

SNAa-D2 (hereinafter, referred to as "D2") and SNAa-sU2 (hereinafter, referred to as "sU2") were synthesized by an automatic DNA synthesizer (ABI-3400 DNA Synthesizer, Applied Biosystems) using an amidite SNA monomer of a natural nucleobase, an amidite SNA monomer of 2,6-diaminopurine (compound 8 of Synthesis Example 1), and an amidite SNA monomer of 2-thiouracil. When using an amidite SNA monomer of 2-thiouracil, a decomposition reaction occurs when a normal oxidizing agent (iodine) is used in the phosphorus oxidation process of the phosphoramidite method. For this reason, tert-butyl hydroperoxide was used as an oxidizing agent. For deprotection of a protecting group removable by an acid on the amidite SNA monomer, a CPG-binding SNA polynucleotide was incubated with TFA (13.9% m-cresol and 2.8% H₂O) at room temperature for 3 hours. Then, the CPG-binding SNA polynucleotide was washed. After drying, cleavage and deprotection were performed at 55°C for 4 to 6 hours in a 28% NH₄OH solution. Next, SNA was purified by reversed-phase HPLC (Kanto Chemical, Mightysil RP-18 GPII column).

Table 1 shows the results of MALDI-TOF MS analysis of the synthesized SNA polynucleotides. No peak for lack of deprotection was observed.

**Table 1**

| | **Found** | **Calcd. [M+H⁺]** |
|---|---|---|
| **SNAa-D2** | **2558.14** | **2558.58** |
| **SNAa-sU2** | **2561.49** | **2562.50** |

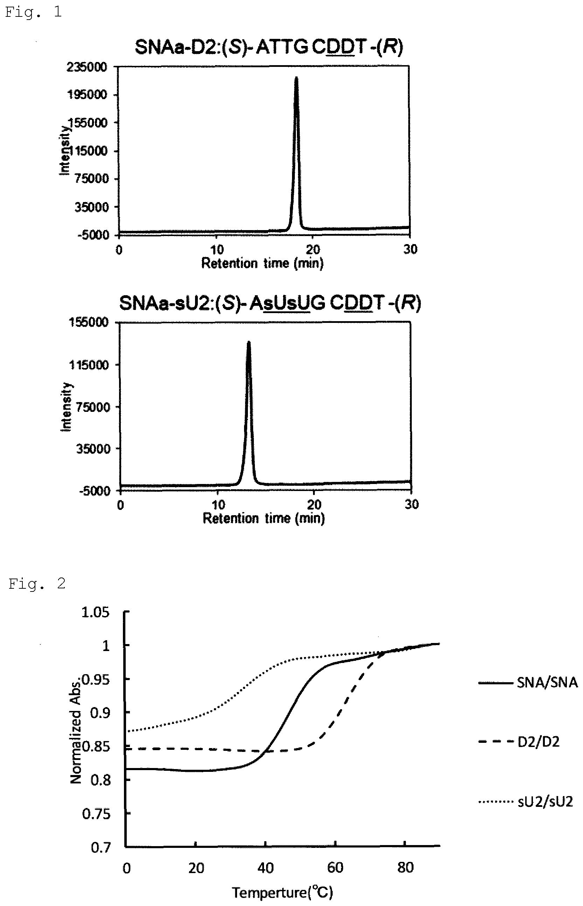

Fig. 1 shows the HPLC profiles of the synthesized SNA polynucleotides.

### Test Example 1: Melting Temperature Measurement 1

The SNA polynucleotides (SNAa, D2, and sU2) were each subjected to melting temperature measurement. Specifically, the measurement was performed in the following manner. Each SNA polynucleotide was dissolved in a 10 mM sodium phosphate buffer (pH: 7.0) containing 100 mM NaCl (final concentration: 2 µM). The solution was analyzed by a Shimadzu UV-1800, and a melting curve was obtained by measuring the change in absorbance at 260 nm as a function of temperature. The melting curve was measured at a temperature ramp of 1.0°C min⁻¹. Tm values were determined from the maximum value of the first derivative of the melting curve.

Fig. 2 shows the obtained melting curves. Further, Table 2 shows the Tm values.

**Table 2**

| **Homo Dimer** | |
|---|---|
| **SNAa** | **46.0** |
| **D2** | **61.0** |
| **sU2** | **30.6** |

A comparison of D2 and sU2 revealed that the melting temperature of Homo Dimer could be significantly reduced by introducing sU into the complementary area of D.

### Test Example 2: Melting Temperature Measurement 2

The SNA polynucleotides (SNAa, D2, and sU2) and a complementary RNA polynucleotide (RNAb) were subjected to melting temperature measurement. The measurement was performed in the same manner as in Test Example 1, except that each SNA polynucleotide and the RNA polynucleotide were dissolved in a 10 mM sodium phosphate buffer (pH: 7.0) containing 100 mM NaCl (SNA polynucleotide final concentration: 2 µM, RNA polynucleotide final concentration: 2 µM).

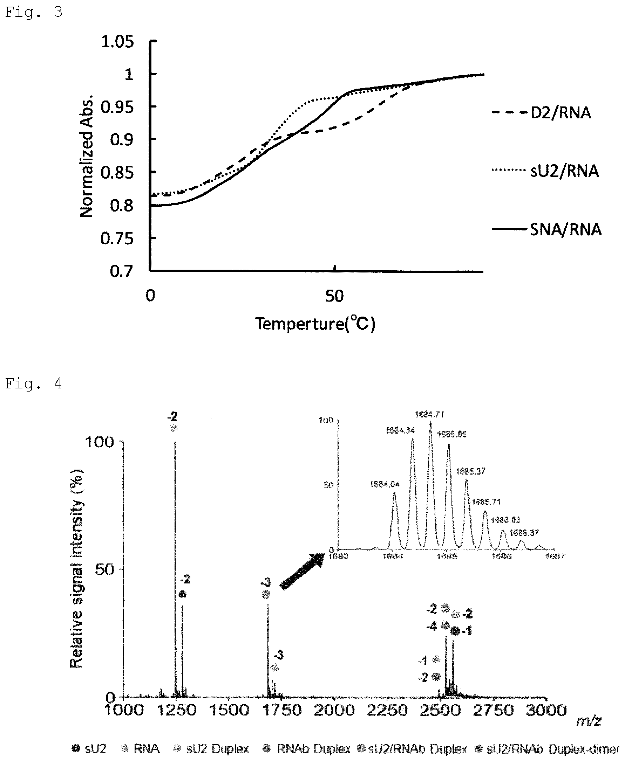

Fig. 3 shows the obtained melting curves. Further, Table 3 shows the Tm values.

**Table 3**

| | **RNAb** | |
|---|---|---|
| | Low temperature | High temperature |
| **SNAa** | **26.1** | **44.0** |
| **D2** | **25.3** | **62.1** |
| **sU2** | **32.7** | - |

Two sigmoid curves were observed for D2/RNAb, and the D2 strand pair and the RNAb pair each formed a duplex; however, sU2/RNAb showed one sigmoid curve, and its Tm was higher than that of sU2/sU2 duplex (30.6°C). This suggested that sU2/RNAb formed a duplex.

In order to confirm this, a sU2/RNAb sample was analyzed by Native MS. Specifically, the analysis was performed in the following manner. The buffer was exchanged to 100 mM triethylammonium acetate (pH: 7.0) by passing the sU2/RNAb sample through a Bio-Rad Micro Bio-Spin 6 column. The buffer-exchanged sample was immediately analyzed by nanoflow electrospray ionization mass spectrometry using gold-coated glass capillaries (approximately 2 to 5 µL of the sample was loaded per analysis). Spectra were recorded on a Waters SYNAPT G2-Si HDMS mass spectrometer in negative ionization mode at 1.33 kV with a sampling cone voltage of 150 V and source offset voltage, 4 V trap and 2 V transfer collision energy, and a trap gas flow rate of 5 mL/min. The spectra were calibrated using 1 mg/mL cesium iodide and analyzed using MassLynx software (Waters).

Fig. 4 shows the obtained Native MS profile. It was confirmed that sU2/RNAb formed a duplex.

In order to quantitatively clarify that sU2/RNAb formed a duplex, analysis was performed by polyacrylamide gel electrophoresis (PAGE). Specifically, the analysis was performed in the following manner. 25 µM of RNAb, SNAa-sU2, or a mixture of RNAb and SNAa-sU2 in a 10 mM phosphate buffer (pH: 7.0) containing 100 mM NaCl was heated to 80°C and then cooled to 4°C. A bromophenol blue-free loading buffer was mixed with the sample, and the mixture was analyzed by 20% Native-PAGE with 10% glycerol under 750 CV, 2 hours, 4°C conditions.

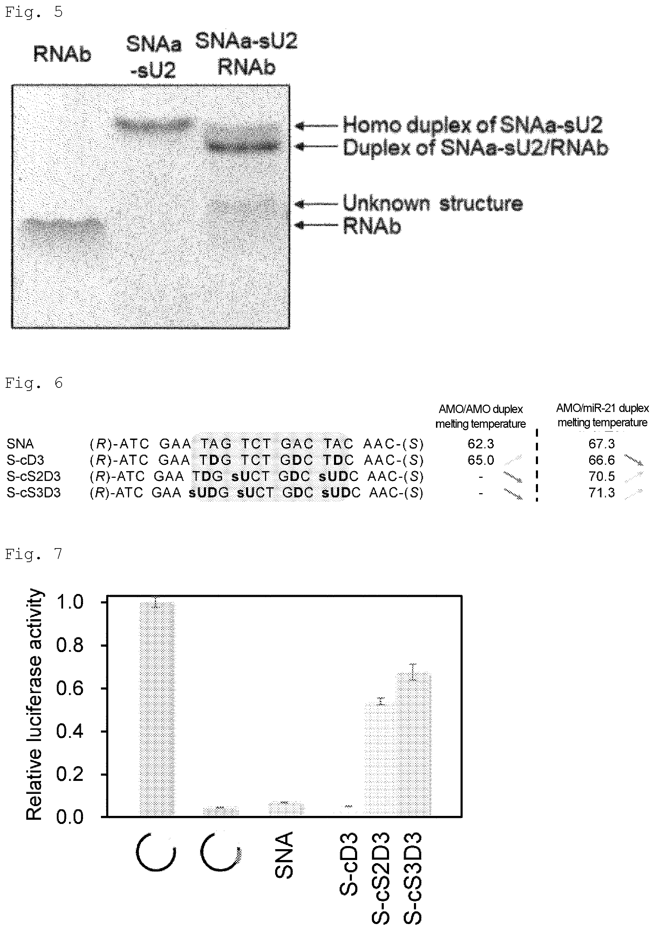

The obtained PAGE was stained with Fast Blast DNA stain (Bio-Rad) and then imaged with an imaging analyzer (FLA9500, GE Healthcare), the results of which are shown in Fig. 5. Since the band obtained by mixing sU2 and RNAb was located at a position clearly different from the band of sU2 or RNAb alone, it was confirmed that sU2 and RNAb formed a duplex. Further, a comparison of the band intensities of sU2 and sU2/RNAb revealed that 90% or more of sU2 formed a duplex with RNAb.

### Synthesis Example 4: Synthesis 2 of Single-Stranded Polynucleotides

Four single-stranded polynucleotides (AMO (anti-miRNA oligonucleotide): SEQ ID NO: 1) comprising a palindromic structure (D represents 2,6-diaminopurine, and sU represents 2-thiouracil) shown in Fig. 6 were prepared in the same manner as in Synthesis Example 3. These are all SNA polynucleotides targeting miRNA-21 (miR-21).

### Test Example 3: Melting Temperature Measurement 3

The Tm values of the polynucleotides of Synthesis Example 4 were determined in the same manner as in Test Example 1. Fig. 6 shows the Tm values. The introduction of the D-sU pair suppressed Homo duplex formation of SNA-AMO and improved RNA recognition ability.

### Test Example 4: Antisense Activity Measurement 1

The antisense activity of the polynucleotides (AMO) of Synthesis Example 4 was measured. Specifically, 40 nM introduction nucleic acid (a reporter plasmid containing a luciferase gene (control) or a reporter plasmid containing a miR21-binding site downstream of a luciferase gene (the expression of luciferase was suppressed by the miR21-binding site), and AMO) was introduced into Hela cells by Lipofectamine 2000, and after incubation for 24 hours, luciferase activity was measured. The improvement of luciferase activity by the introduction of AMO indicates the expression of antisense activity by AMO.

The results are shown in Fig. 7. The introduction of the D-sU pair greatly improved the antisense activity.

### Synthesis Example 5: Synthesis 3 of Single-Stranded Polynucleotides

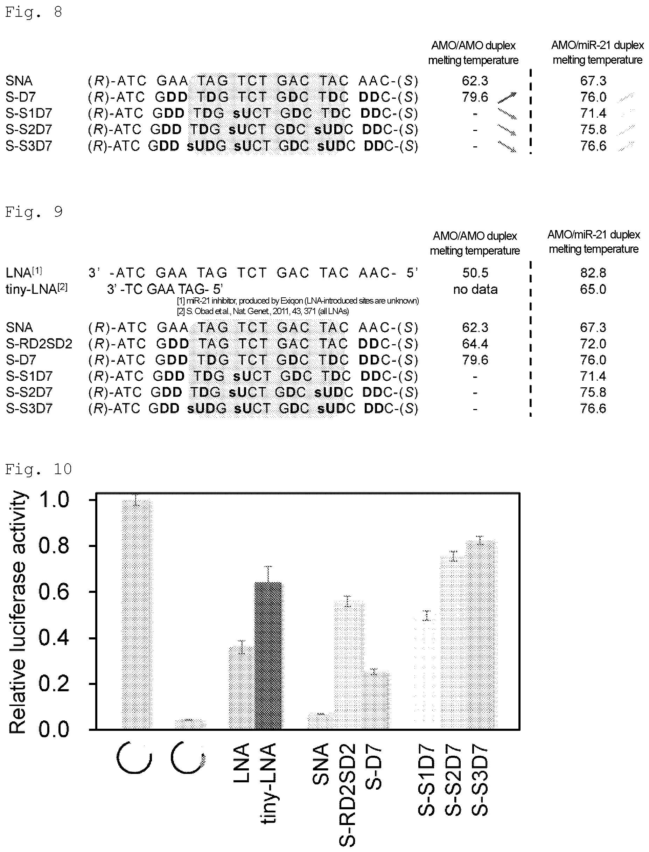

Five single-stranded polynucleotides (AMO: SEQ ID NO: 1) comprising a palindromic structure (D represents 2,6-diaminopurine, and sU represents 2-thiouracil) shown in Fig. 8 were prepared in the same manner as in Synthesis Example 3. These are all SNA polynucleotides targeting miRNA-21 (miR-21).

### Test Example 5: Melting Temperature Measurement 4

The Tm values of the polynucleotides of Synthesis Example 5 were determined in the same manner as in Test Example 1. Fig. 8 shows the Tm values. The introduction of the D-sU pair suppressed Homo duplex formation of SNA-AMO and improved RNA recognition ability.

### Synthesis Example 6: Synthesis 4 of Single-Stranded Polynucleotides

Six SNA single-stranded polynucleotides (AMO: SEQ ID NO: 1) comprising a palindromic structure (D represents 2,6-diaminopurine, and sU represents 2-thiouracil) and two LNA single-stranded polynucleotides (AMO: SEQ ID NO: 1 or a short-chain sequence thereof) shown in Fig. 9 were prepared in the same manner as in Synthesis Example 3.

### Test Example 6: Melting Temperature Measurement 5

The Tm values of the polynucleotides of Synthesis Example 6 were determined in the same manner as in Test Example 1. Fig. 9 shows the Tm values. The introduction of the D-sU pair suppressed Homo duplex formation of SNA-AMO and improved RNA recognition ability.

### Test Example 7: Antisense Activity Measurement 2

The antisense activity of the polynucleotides (AMO) of Synthesis Example 6 was measured in the same manner as in Test Example 5. The results are shown in Fig. 10. The development of D-sU-conjugated SNA-AMO with very high activity succeeded.

### Synthesis Example 7: Synthesis 5 of Single-Stranded Polynucleotides

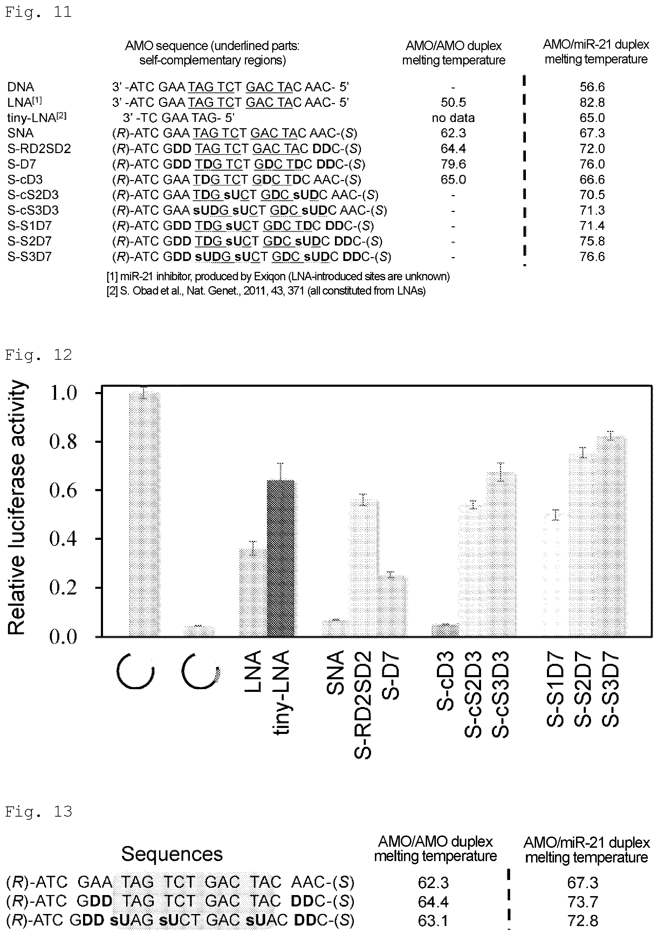

Nine SNA single-stranded polynucleotides (AMO: SEQ ID NO: 1) comprising a palindromic structure (D represents 2,6-diaminopurine, and sU represents 2-thiouracil), two LNA single-stranded polynucleotides (AMO: SEQ ID NO: 1 or a short-chain sequence thereof), and one DNA single-stranded polynucleotide (AMO: SEQ ID NO: 1) shown in Fig. 11 were prepared in the same manner as in Synthesis Example 3.

### Test Example 8: Melting Temperature Measurement 6

The Tm values of the polynucleotides of Synthesis Example 7 were determined in the same manner as in Test Example 1. Fig. 11 shows the Tm values. The introduction of the D-sU pair suppressed Homo duplex formation of SNA-AMO and improved RNA recognition ability.

### Test Example 9: Antisense Activity Measurement 3

The antisense activity of the polynucleotides (AMO) of Synthesis Example 7 was measured in the same manner as in Test Example 5. The results are shown in Fig. 12. The development of D-sU-conjugated SNA-AMO with very high activity succeeded.

### Synthesis Example 8: Synthesis 6 of Single-Stranded Polynucleotides

Three SNA single-stranded polynucleotides (AMO: SEQ ID NO: 1) comprising a palindromic structure (D represents 2,6-diaminopurine, and sU represents 2-thiouracil) shown in Fig. 13 were prepared in the same manner as in Synthesis Example 3.

### Test Example 10: Melting Temperature Measurement 7

The Tm values of the polynucleotides of Synthesis Example 8 were determined in the same manner as in Test Example 1. Fig. 13 shows the Tm values. The introduction of sU alone does not affect the melting temperature.

### Test Example 11: Antisense Activity Measurement 4

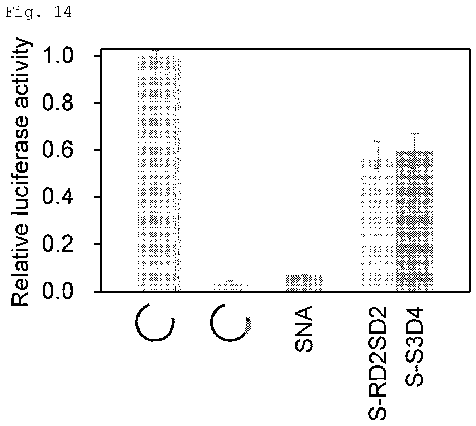

The antisense activity of the polynucleotides (AMO) of Synthesis Example 8 was measured in the same manner as in Test Example 5. The results are shown in Fig. 14. The introduction of sU alone does not affect the antisense activity.

Sequence Listing

## Claims

1. A single-stranded polynucleotide comprising a palindromic structure comprising an acyclic polynucleotide structural unit, wherein adenine and/or an analog thereof in the palindromic structure is replaced by diaminopurine, and thymine and/or an analog thereof at a position complementary to the adenine is replaced by a thiouracil derivative.

2. The single-stranded polynucleotide according to claim 1, wherein the acyclic polynucleotide structural unit is represented by formula (1): 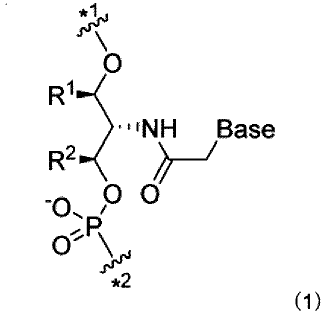 wherein R¹ and R² are the same or different, and each is a hydrogen atom or an organic group, excluding a case where R¹ and R² are both organic groups, and Base is a nucleobase.

3. The single-stranded polynucleotide according to claim 2, wherein R¹ is a hydrogen atom or a chain hydrocarbon group, and R² is a hydrogen atom.

4. The single-stranded polynucleotide according to any one of claims 1 to 3, wherein the palindromic structure is a single-stranded polynucleotide consisting of a base sequence represented by formula (2): A¹-B-A², wherein the base length of A¹ and A² is 3 or more, A¹ and A² are base sequences complementary to each other, and the base length of B is an integer of 0 to (base length of A¹ + base length of A²)/4).

5. The single-stranded polynucleotide according to any one of claims 1 to 4, wherein the palindromic structure consists of the acyclic polynucleotide structural unit.

6. The single-stranded polynucleotide according to any one of claims 1 to 5, wherein the number of diaminopurines and the number of thiouracil derivatives are each 2 or more.

7. The single-stranded polynucleotide according to any one of claims 1 to 6, which has a base length of 6 to 500.

8. The single-stranded polynucleotide according to any one of claims 1 to 7, which is complementary to a cellular endogenous polynucleotide.

9. The single-stranded polynucleotide according to any one of claims 1 to 8, which is anti-miRNA or an anti-mRNA polynucleotide.

10. A double-stranded polynucleotide comprising the single-stranded polynucleotide according to any one of claims 1 to 9.

11. A compound or a salt thereof or a solvate thereof, the compound being represented by formula (3): 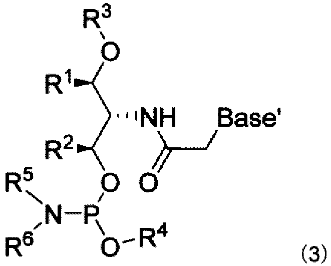 wherein R¹ and R² are the same or different, and each is a hydrogen atom or an organic group, excluding a case where R¹ and R² are both organic groups, R³ and R⁴ are the same or different, and each is a protecting group for a hydroxyl group, R⁵ and R⁶ are the same or different, and each is an alkyl group, and Base' is a nucleobase in which an amino group of diaminopurine is protected by a protecting group removable by an acid, or a nucleobase in which a thiocarbonyl group of a thiouracil derivative is protected by a protecting group removable by an acid.

12. The compound or a salt thereof or a solvate thereof according to claim 11, wherein the compound is represented by formula (3A) or (3B): 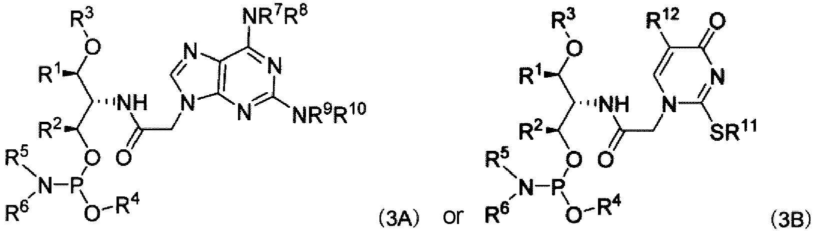 wherein R¹, R²/R³, R⁴, R⁵, and R⁶ are as defined above, R⁷, R⁸, R⁹, R¹⁰, and R¹¹ are the same or different, and each is a protecting group removable by an acid, and R¹² is a hydrogen atom or an alkyl group.

13. The compound or a salt thereof or a solvate thereof according to claim 12, wherein R⁷, R⁸, R⁹, and R¹⁰ are Boc groups, and R⁹ is an MMPM group.

14. The compound or a salt thereof or a solvate thereof according to any one of claims 11 to 13, wherein R³ is a group represented by formula (4): 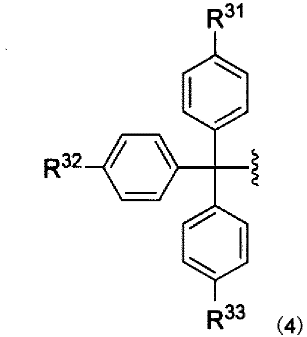 wherein R³¹, R³², and R³³ are the same or different, and each is a hydrogen atom or an alkoxy group;
R⁴ is -(CH₂)₂-CN; and
R⁵ and R⁶ are isopropyl groups.

15. A method for producing a single-stranded polynucleotide by a phosphoramidite method using the compound or a salt thereof or a solvate thereof according to any one of claims 11 to 14 as an amidite monomer.
